# FASCICULE DE BREVET EUROPEEN

(11) **EP 3 164 179 B1**
(45) Date de publication et mention de la délivrance du brevet: **02.09.2020**
(21) Numéro de dépôt: 15753090.8
(22) Date de dépôt: 02.07.2015
(51) Int. Cl.: A61M 5/20, A61M 5/315

(54) **AUTOINJECTEUR**
AUTOINJEKTOR
AUTOINJECTOR

(30) Priorité: 04.07.2014 FR 1456432
(43) Date de publication de la demande: 10.05.2017
(73) Titulaire: APTAR France SAS, 27110 Le Neubourg (FR)
(72) Inventeur: FABIEN, David, 22700 Saint Quay Perros (FR)
(74) Mandataire: CAPRI
(86) Numéro de dépôt international: PCT/FR2015/051827
(87) Numéro de publication internationale: WO 2016/001592

(56) Documents cités:
- EP-A1- 2 705 862
- WO-A1-2011/043714
- WO-A1-2011/101382
- WO-A1-2015/001280
- WO-A2-2009/010591
- US-A1- 2005 277 886

## Description

La présente invention concerne un autoinjecteur.

Les autoinjecteurs sont bien connus dans l'état de la technique. Ces dispositifs ont principalement pour but de réaliser une injection automatique du contenu d'une seringue à l'intérieur du corps d'un patient. Divers systèmes existent pour rendre automatique la pénétration de l'aiguille dans le corps du patient ainsi que l'injection du produit fluide contenu dans la seringue. Les autoinjecteurs sont des dispositifs relativement complexes qui doivent répondre à un certain nombre d'exigences de contraintes pour être fiables. La robustesse du dispositif, sa maniabilité, et sa facilité d'utilisation pour l'utilisateur sont également des éléments importants. Par ailleurs, la plupart de ces autoinjecteurs étant à usage unique, le coût de fabrication et d'assemblage est également un facteur dont il faut tenir compte.

Il existe de nombreux autoinjecteurs sur le marché, qui présentent toutefois tous un certain nombre d'inconvénients.

Ainsi, pour les autoinjecteurs qui utilisent le même ressort pour d'abord effectuer le piquage puis l'injection, le ressort doit être suffisamment puissant pour garantir la totalité de la phase d'injection. Ceci est d'autant plus vrai qu'au début de la phase d'injection, il faut généralement une force relativement importante pour décoller le piston de la seringue. De ce fait, le ressort exprime sa puissance maximale lors du piquage, ce qui peut rendre cette phase de piquage douloureuse. De plus, avec un tel ressort très puissant lors du piquage, il existe un risque important de casse de la collerette de la seringue, en particulier quand il s'agit de seringue en verre.

Par ailleurs, il peut être souhaitable d'avoir une indication visuelle et/ou sonore pour indiquer à l'utilisateur que l'injection est terminée.

WO2017/043714, WO2009/010591 et WO2011/101382 révèlent la présence d'un autoinjecteur.

La présente invention a pour but de fournir un autoinjecteur qui ne reproduit pas les inconvénients susmentionnés, et qui permet de répondre aux différentes exigences et contraintes importantes pour une utilisation sûre et fiable de l'autoinjecteur.

La présente invention a aussi pour but de fournir un autoinjecteur qui soit fiable et sûr d'utilisation, qui permette de garantir la distribution de la totalité du produit fluide à l'endroit souhaité, qui informe de manière fiable l'utilisateur de la fin d'injection et qui soit simple et peu coûteux à fabriquer et à assembler.

La présente invention a donc pour objet un autoinjecteur comportant un corps, un réservoir contenant du produit fluide et comportant un piston et une aiguille, tel qu'une seringue pré-remplie, ledit autoinjecteur comportant une tige de piston adaptée à coopérer avec le piston dudit réservoir, ladite tige de piston étant déplaçable entre une position de repos et une position d'injection dans laquelle ladite tige de piston a déplacé le piston du réservoir pour injecter le produit fluide à travers l'aiguille, un ressort d'actionnement étant prévu pour solliciter ladite tige de piston vers sa position d'injection, l'autoinjecteur comprenant un système de réglage de force adapté à exercer au moins une force F2 sur ladite tige de piston, ladite force F2 s'ajoutant à la force exercée par ledit ressort d'actionnement sur ladite tige de piston en début d'injection pour amplifier la force exercée sur ledit piston par ladite tige de piston en début d'injection, ledit système de réglage de force comprenant deux organes pivotants coopérant avec ladite tige de piston, lesdits organes pivotants étant reliés entre eux par deux éléments élastiques, ledit corps comportant un manchon, lesdits organes pivotants étant monté pivotants sur ledit manchon autour d'axes fixes, ledit autoinjecteur comprend un indicateur visuel et/ou sonore adapté à indiquer à l'utilisateur la fin de l'injection, ledit indicateur étant formé par et/ou fixé sur ledit manchon et comportant une partie d'indication déplaçable et/ou déformable par rapport audit corps pour coopérer, après la fin de l'injection, avec au moins une fenêtre de visualisation dudit autoinjecteur, lesdit éléments élastique sont fixés auxdits organes pivotants au niveau d'axes mobiles parallèles, tels que des tiges avant deux bords latéraux formés sur lesdits organes pivotants, dans lequel, lorsque la tige de piston se déplace vers sa position d'injection, lesdits axes mobiles sont disposés en arrière desdits axes fixes dans le sens de déplacement de ladite tige de piston, lesdits éléments élastiques chargés, en début de déplacement de la tige de piston vers sa position d'injection, faisant tourner lesdits organes pivotants de telle manière à détendre lesdits éléments élastiques, créant ainsi une force d'amplification F2 en début d'injection.

Avantageusement, chaque organe pivotant comprend plusieurs projections adaptées à coopérer avec plusieurs projections radiales de la tige de piston.

Avantageusement, lors du piquage, la tige de piston coopère avec le piston du réservoir pour déplacer ledit réservoir par rapport au corps.

Avantageusement, avant injection, ladite tige de piston est d'abord déplacée par ledit ressort d'actionnement entre ladite position de repos et une position de piquage, dans laquelle ladite tige de piston a déplacé ledit réservoir par rapport audit corps pour réaliser le piquage.

Avantageusement, lors du piquage, la tige de piston coopère avec le piston du réservoir pour déplacer ledit réservoir par rapport au corps.

Avantageusement, lorsque la tige de piston se déplace de sa position de repos vers sa position de piquage, lesdits axes mobiles sont disposés en avant desdits axes fixes dans le sens de déplacement de ladite tige de piston, ladite tige de piston, en fin de déplacement vers sa position de piquage, faisant tourner lesdits organes pivotants de telle manière à charger lesdits éléments élastiques, créant ainsi une force de freinage F1 en fin de piquage, ladite force F1 étant opposée à la force exercée par ledit ressort d'actionnement sur ladite tige de piston en fin de piquage pour diminuer la force exercée sur ledit réservoir par ladite tige de piston en fin de piquage.

Avantageusement, ledit autoinjecteur est actionné par un bouton axial.

Avantageusement, ladite au moins une fenêtre de visualisation est formée dans ledit bouton axial.

Avantageusement, ledit bouton axial comporte une seule fenêtre de visualisation disposée dans une paroi d'extrémité axiale inclinée dudit bouton axial.

En variante, ledit bouton axial comporte une pluralité de fenêtres de visualisation, réparties autour du bord d'extrémité axial extérieur dudit bouton axial.

Selon un autre mode de réalisation avantageux, ladite au moins une fenêtre de visualisation est formée dans ledit corps.

Avantageusement, ledit indicateur est formé par ledit manchon comportant une projection axiale monobloc formant ladite partie d'indication de l'indicateur.

En variante, ledit indicateur est formé par un élément d'indication pivotant monté pivotant sur ledit manchon, une partie d'extrémité dudit élément d'indication pivotant formant ladite partie d'indication de l'indicateur.

Avantageusement, ledit élément d'indication pivotant est entrainé en rotation par lesdits organes pivotants, en fin d'injection.

Avantageusement, ledit manchon comporte au moins une patte axiale radialement déformable et coopérant avec ledit corps, la déformation de ladite patte axiale étant bloquée par ladite tige de piston avant et pendant le déplacement de ladite tige de piston, bloquant ainsi tout déplacement axial dudit manchon par rapport audit corps, ladite tige de piston libérant ledit blocage en fin d'injection, de sorte que ladite patte axiale se déforme radialement vers l'intérieur, permettant le déplacement axial dudit manchon par rapport audit corps pour réaliser une indication visuelle et/ou sonore.

Ces caractéristiques et avantages et d'autres de la présente invention apparaîtront plus clairement au cours de la description détaillée suivante, faite en référence aux dessins joints, donnés à titre d'exemples non limitatifs, et sur lesquels :
La figure 1 est une vue schématique en perspective éclatée des composants d'un autoinjecteur, selon un mode de réalisation avantageux,
La figure 2 est une vue de détail en section transversale du dispositif de la figure 1,
La figure 3 est une vue de détail de côté d'une partie du dispositif de la figure 1,
La figure 4 est une vue similaire à celle de la figure 3, vue de dessous,
Les figures 5 à 9 illustrent de manière schématique, vues en section transversale, les séquences successives de la présente invention, selon le mode de réalisation de la figure 1,
Les figures 10 à 14 illustrent de manière schématique, vues de côté, les séquences successives des figures 5 à 9,
Les figures 15 à 17 illustrent de manière schématique, vues en section transversale, un indicateur visuel et/ou sonore selon un premier mode de réalisation de l'invention,
Les figures 18 à 20 illustrent de manière schématique, vues de côté, l'indicateur des figures 15 à 17,
La figure 21 est une vue en perspective du manchon selon une mode de réalisation avantageux de la présente invention,
La figure 22 est une vue en perspective découpée du manchon de la figure 21,
Les figures 23 à 25 illustrent trois autres modes de réalisation d'un indicateur visuel et/ou sonore, en position de repos,
Les figures 26 à 28 sont des vues similaires à celles des figures 15 à 17, illustrant encore un autre mode de réalisation de la présente invention, et
Les figures 29 à 32 illustrent en section transversale encore un autre mode de réalisation de l'invention.

L'autoinjecteur va être décrit ci-après en référence à plusieurs modes de réalisation avantageux de celui-ci. Il est toutefois à noter que les autoinjecteurs, qui sont des appareils complexes, comprennent plusieurs modules pour réaliser plusieurs fonctions. Ces divers modules peuvent être utilisés séparément et indépendamment les uns des autres, sans être nécessairement combinés aux autres modules, et pourraient notamment être utilisés dans des autoinjecteurs de forme différente de celle représentée sur les dessins. De plus, il est à noter que les dessins sont des vues schématiques qui ne représentent pas forcément la forme exacte des composants d'un autoinjecteur, et ne sont pas forcément à l'échelle, notamment dans des buts de clarté. Par ailleurs, les dessins ne représentent pas forcément tous les éléments constitutifs d'un autoinjecteur, mais seulement les éléments nécessaires au fonctionnement de la présente invention. Ainsi, divers éléments et modules supplémentaires et/ou complémentaires pourraient être associés à l'autoinjecteur représenté sur les figures.

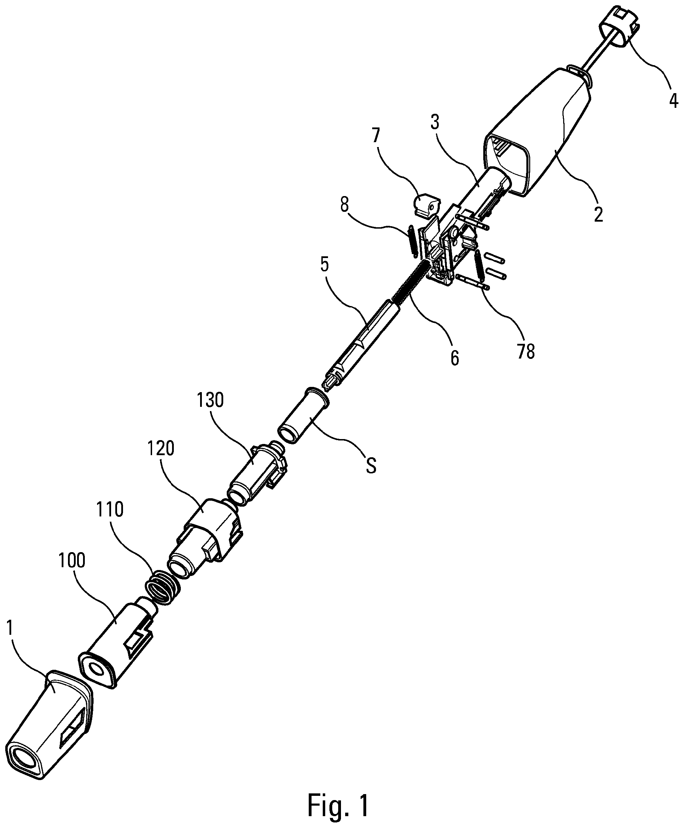

En se référant à la figure 1, les différents composants de l'autoinjecteur, selon un mode de réalisation avantageux, sont représentés de manière éclatée.

Dans l'ordre des références numériques, l'autoinjecteur comporte un corps inférieur 1, un corps supérieur 2 contenant un manchon 3, un bouton axial d'actionnement 4, une tige de piston 5, un ressort d'actionnement 6, et deux organes pivotants 7, interconnectés au moyen de deux éléments élastiques 8, de préférence sous forme de ressorts.

Il est à noter que les corps inférieur et supérieur pourraient être remplacés par un corps unique. De même, un corps constitué de plus de deux parties de corps est aussi envisageable.

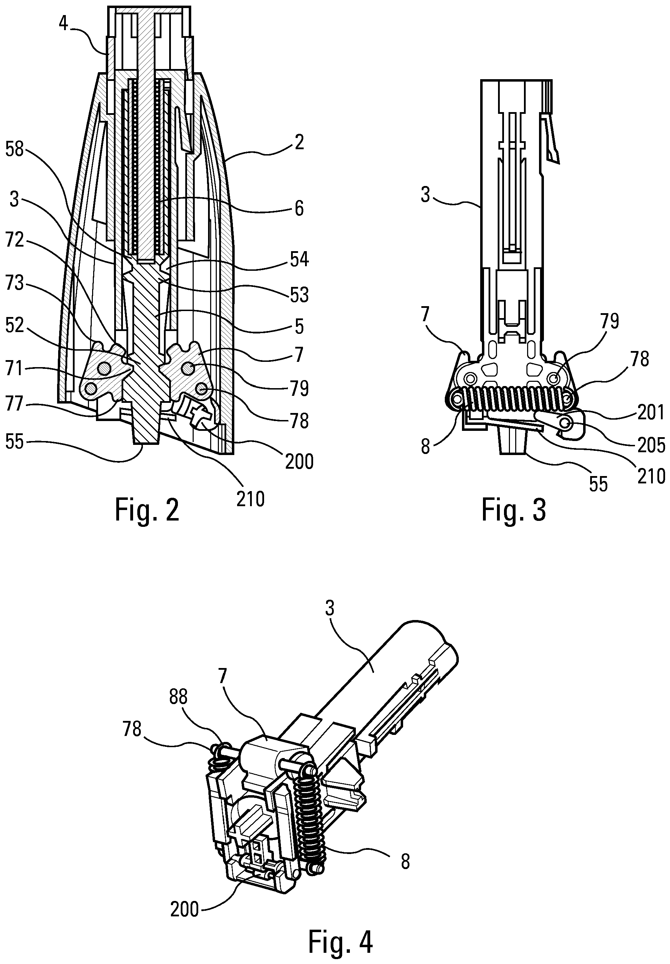

Le manchon 3 peut être encliqueté dans le corps supérieur 2 au moyen d'une patte d'encliquetage 33, visible notamment sur les figures 2 et 3.

Un réservoir S est inséré dans ledit autoinjecteur, notamment dans son corps inférieur 1. Ce réservoir S contient du produit fluide, et comporte un piston et une aiguille (non représentés dans ce mode de réalisation). Le piston est adapté à se déplacer dans ledit réservoir S pour injecter le produit fluide à travers ladite aiguille.

La présente description sera faite en référence à une seringue S, qui peut être de tout type. Plus généralement, il est entendu que le terme « seringue » dans la présente description englobe tout type de réservoir associé à une aiguille. De préférence, le réservoir S est une seringue pré-remplie.

Le corps inférieur 1 comporte à son extrémité avant (dans le sens de déplacement de la seringue S) une ouverture permettant le passage de l'aiguille lors de la phase de piquage.

Le corps inférieur 1 contient un manchon actionneur 100 dont la surface d'extrémité axiale 105 est en contact avec la partie du corps de l'utilisateur où l'injection doit se faire. Après actionnement, le manchon actionneur 100 recouvre l'aiguille de la seringue S pour éviter tout risque de piqure avec ladite aiguille. Le manchon actionneur 100 est adapté à coulisser par rapport audit corps inférieur 1 entre une position initiale de repos, avant actionnement, dans laquelle il se projette axialement hors dudit corps inférieur 1, une position actionnée, dans laquelle il est déplacé axialement vers l'intérieur dudit corps inférieur 1, et une position finale de sécurité, dans laquelle il est à nouveau projeté hors dudit corps inférieur 1, pour recouvrir l'aiguille de la seringue S après injection. Il est à noter que la position finale de sécurité peut être identique à la position initiale de repos, ou en variante, ces deux positions peuvent être différentes, avec par exemple le manchon actionneur 100 s'étendant axialement plus loin hors dudit corps inférieur 1 dans ladite position finale de sécurité par rapport à ladite position initiale de repos. Ce manchon actionneur 100 est avantageusement sollicité axialement vers l'extérieur dudit corps inférieur 1 par un ressort 110.

Le corps inférieur 1 peut en outre contenir un corps interne 120 pouvant recevoir un élément de support de réservoir 130 dans lequel est insérée ladite seringue S.

Le corps supérieur 2 se fixe au corps inférieur 1, et il peut recevoir un manchon central 3 adapté à loger la tige de piston 5 et le ressort 6.

Le bouton axial d'actionnement 4 peut être monté coulissant axialement par rapport au corps supérieur 2, et en contact avec la tige de piston 5. Ainsi, en appuyant sur le bouton axial 4 pour l'enfoncer axialement dans le corps supérieur 2, on déplace axialement la tige de piston 5, ce qui permet l'actionnement du dispositif comme cela sera décrit ci-après. Le bouton axial pourrait en variante être remplacé par un bouton latéral.

La tige de piston 5 comporte une partie arrière 54 et une extrémité avant 55, dans le sens de déplacement de la tige de piston 5 dans le corps supérieur 2.

Dans cet exemple, la partie arrière 54 définit une partie tubulaire recevant le ressort 6 et une partie du bouton axial d'actionnement 4.

L'extrémité avant 55 a pour but de contacter le piston de la seringue S pour déplacer ledit piston et ainsi injecter le produit contenu dans la seringue S à travers l'aiguille.

La tige de piston 5 peut aussi comporter plusieurs projections radiales 52, 53. Une première projection radiale 52, proximal de l'extrémité avant 55, qui définit une surface avant 51 formant épaulement. Et une seconde projection radiale 53, décalée axialement de ladite première projection radiale 52 en direction de l'extrémité arrière 54, et qui définit une surface avant et une surface arrière. Bien sûr, il ne s'agit là que d'exemples de réalisation, et un homme du métier est capable de réaliser ces épaulements et projections radiales d'une manière différente de celle représentée sur les dessins. En particulier, lesdites projections radiales 52, 53 ne sont pas nécessairement dans la partie avant de la tige de piston 5, comme représenté sur les dessins, mais elles pourraient être réalisées sur une autre partie de la tige de piston 5. Des creux pourraient aussi remplacer les projections.

Le ressort d'actionnement 6 peut s'appuier d'une part dans le manchon 3 et d'autre part sur la tige de piston, par exemple sur un quatrième épaulement 58 décalé axialement dudit troisième épaulement 53 en direction de la partie arrière 54. Dans l'exemple représenté, ce quatrième épaulement 58 forme une base de la partie tubulaire susmentionnée.

Les organes pivotants 7 sont avantageusement assemblés de manière pivotante sur le manchon 3, et ils sont avantageusement identiques. Ils sont de préférence disposés de part et d'autre de la tige de piston 5. Ils ne sont pas déplaçables axialement par rapport audit corps inférieur 1, mais seulement en pivotement autour de leurs axes de rotation 79 qui sont parallèles. En variante, ils pourraient être assemblés différemment, notamment sur le corps inférieur 1 ou sur le corps supérieur 2.

Chaque organe pivotant 7 peut comporter plusieurs projections 71, 72, 73. Une première projection 71 adaptée à coopérer avec la surface avant (dans le sens de déplacement axial de la tige de piston au cours de l'actionnement) de la première projection radiale 52 de la tige de piston 5. Une seconde projection 72 est adaptée à coopérer avec la surface avant de la seconde projection radiale 53. Et une troisième projection 73 est adaptée à coopérer avec la surface arrière de la seconde projection radiale 53. Bien entendu, d'autres mises en œuvre sont envisageables, par exemple avec un nombre différent de projections. Des creux pourraient aussi remplacer les projections.

Les éléments élastiques 8 relient les deux organes pivotants 7 entre eux. Le principe consiste à combiner deux axes de rotation fixes, en particulier les axes de rotation 79 des deux organes pivotants 7, avec deux axes mobiles, en particulier les points d'accroche 78 des organes pivotants 7 sur lesquels se fixent les éléments élastiques 8.

Ainsi, comme visible sur la figure 4, le premier élément élastique 8 peut se fixer d'une part sur un premier point d'accroche du premier organe pivotant et d'autre part sur un premier point d'accroche du second organe pivotant, et il peut en être de même et de manière symétrique pour l'autre élément élastique. De préférence, chaque organe pivotant 7 comporte une tige 78 ayant deux bords latéraux saillants. Le premier élément élastique relie alors les premiers bords saillants entre eux et le second élément élastique relie les seconds bords saillants entre eux. D'autres variantes de réalisation sont possibles. Avantageusement, lorsque les éléments élastiques 8 sont des ressorts, ils sont identiques et comportent des œillets 88 adaptés à se fixer sur lesdits premiers et seconds bords saillants 78 des organes pivotants 7. En variante, les éléments élastiques pourraient être différents, par exemple sous la forme de joints toriques ou d'autres éléments en matériau élastiquement déformable. L'utilisation d'anneaux en matériau élastique, tels que de joints toriques, en remplacement des ressorts illustrés sur les dessins permettrait notamment de réduire l'encombrement radial du système.

Ainsi, lorsque les axes mobiles 78 sont décalés axialement par rapport aux axes de rotation fixes 79, ils exercent une force par l'intermédiaire des éléments élastiques 8.

Lorsque lesdits axes mobiles 78 sont disposés en avant desdits axes fixes 79 dans le sens de déplacement de la tige de piston 5, cette force va à l'encontre de la rotation impartie auxdits organes pivotants 7 par ladite tige de piston 5. Dans ce cas, la rotation des organes pivotants 7 est donc freinée par lesdits éléments élastiques 8.

Au contraire, lorsque lesdits axes mobiles 78 sont disposés en arrière desdits axes fixes 79 dans le sens de déplacement de la tige de piston 5, cette force va dans le sens de la rotation impartie auxdits organes pivotants 7 par ladite tige de piston 5. Dans ce cas, la rotation des organes pivotants 7 est donc amplifiée par lesdits éléments élastiques 8.

Lorsque les axes fixes 79 et mobiles 78 sont alignés, on se trouve à un point neutre, dans lequel lesdits éléments élastiques 8 n'influencent pas la rotation des organes pivotants 7. C'est dans cette position que le système bascule d'un état de « freinage » ou « d'amortissement » à un état « d'amplification ».

Un tel système de réglage de force est adapté à exercer une force F1 et/ou une force F2 sur ladite tige de piston 5. La force F1 est opposée à la force exercée par le ressort d'actionnement 6 sur la tige de piston 5 en fin de piquage pour diminuer la force exercée sur ledit réservoir S par ladite tige de piston 5 en fin de piquage. La force F2 s'ajoute au contraire à la force exercée par le ressort d'actionnement 6 sur la tige de piston 5 en début d'injection pour amplifier la force exercée sur ledit piston P par ladite tige de piston 5 en début d'injection. Le système de réglage de force peut exercer seulement la force F1, seulement la force F2, ou les deux forces F1 et F2.

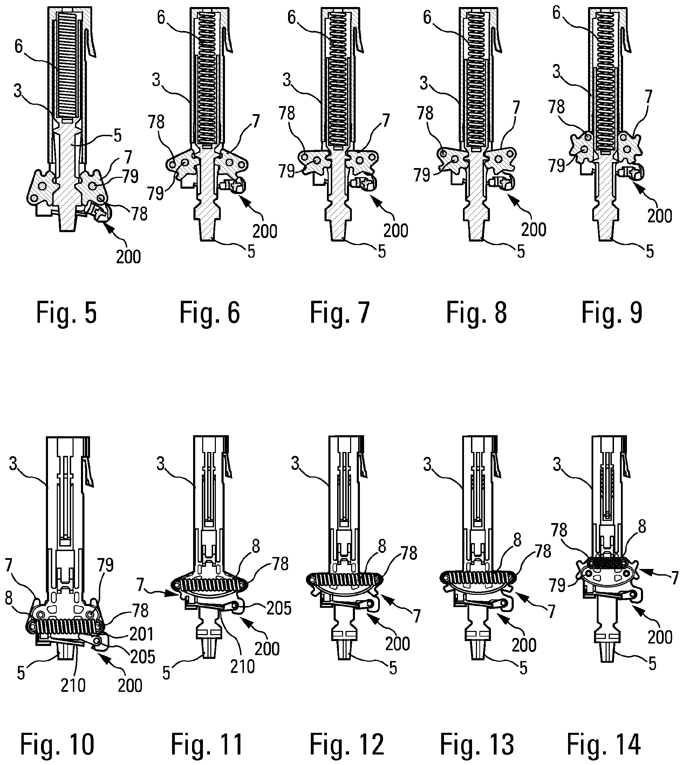

Les figures 5 à 14 illustrent les séquences d'actionnement de l'autoinjecteur de la figure 1.

Sur les figures 5 et 10, l'autoinjecteur est en position de repos avant actionnement. Dans cette position de repos, l'aiguille de la seringue S est disposée à l'intérieur du corps inférieur 1. Lesdits axes mobiles 78 sont disposés en avant desdits axes fixes 79 dans le sens de déplacement de la tige de piston 5, Le ressort d'actionnement 6 sollicite la tige de piston 5 en direction du piston de la seringue S, mais la tige de piston 5 est retenue dans la position de repos par la première projection 71 des organes pivotants 7 qui coopère avec le premier épaulement 51 de la tige de piston 5. Sous la pression du ressort d'actionnement 6, ledit premier épaulement 51 de la tige de piston 5 pousse la première projection 71 des organes pivotants 7 en rotation, mais cette rotation est bloquée par un système de verrouillage 200.

Ce système de verrouillage 200 peut comporter au moins un élément de verrouillage déplaçable et/ou déformable entre une position de verrouillage, dans laquelle il bloque au moins un organe pivotant 7 en rotation, et une position de déverrouillage, dans laquelle ledit au moins un organe pivotant 7 peut pivoter. Ledit élément de verrouillage 201 est sollicité élastiquement vers sa position de verrouillage par un organe de sollicitation 210, tel qu'une lame élastique. Avantageusement, ledit élément de verrouillage 201 est monté pivotant sur ledit manchon 3 dudit corps, autour d'un axe de rotation 205. Dans l'exemple représenté, ledit élément de verrouillage 201 comporte une partie de blocage 202, qui coopère directement avec un organe pivotant 7, et une partie de commande 203 qui coopère avec un élément de déverrouillage 101 solidaire du manchon actionneur 100. De préférence, ledit élément de déverrouillage 101 est formé par une projection axiale dudit manchon actionneur 100. L'élément de déverrouillage pourrait être réalisé différemment.

Lorsque l'utilisateur veut utiliser l'autoinjecteur, il prend le dispositif, par exemple au niveau du corps supérieur 2 et il appuie le manchon actionneur 100 contre la partie de son corps où il veut réaliser l'injection. Le manchon actionneur 100 va alors se déplacer axialement vers l'intérieur du corps inférieur 1. Lorsque la course dudit manchon actionneur 100 vers l'intérieur du corps inférieur 1 est suffisante pour permettre la coopération entre l'élément de verrouillage 201 et l'élément de déverrouillage 101, l'élément de déverrouillage 101 va faire pivoter l'élément de verrouillage 201 du système de verrouillage autour de son axe 205, ce qui libère les organes pivotants 7. Si l'utilisateur relâche la pression sur le manchon actionneur 100 sans actionner l'autoinjecteur via le bouton d'actionnement 4, la lame élastique 210 va ramener l'élément de verrouillage 201 en position de verrouillage. Ceci permet notamment à l'utilisateur de choisir le site d'injection en testant plusieurs endroits sans actionner le bouton d'actionnement 4. Lorsque, après avoir déverrouillé les organes pivotants 7 par appui sur le manchon actionneur 100, l'utilisateur appuie sur le bouton axial d'actionnement 4, il déplace légèrement la tige de piston 5 axialement, ce qui provoque la rotation des organes pivotants 7 et l'actionnement de l'autoinjecteur.

Avantageusement, ledit élément de déverrouillage 101 coopère avec ledit système de verrouillage 200 en fin de course dudit manchon actionneur 100 entre sa position initiale de repos et sa position actionnée, notamment lorsque ledit manchon actionneur 100 a effectué au moins 90% de ladite course. Ceci permet de libérer l'actionnement de l'autoinjecteur seulement lorsqu'on est certain que le produit sera expulsé à la profondeur d'injection souhaitée, et d'éviter ainsi le risque d'un actionnement prématuré à une trop faible profondeur.

La rotation des organes pivotants 7 générée par la force d'actionnement provoque le désengrènement entre la première projection 71 et le premier épaulement 51. Ceci libère donc la tige de piston 5, qui est alors déplacée axialement sous l'effet du ressort d'actionnement 6. Ceci provoque le déplacement de la seringue S dans le corps inférieur 1 et donc le piquage.

Lorsque la seconde projection 72 des organes pivotants atteint la surface avant de la seconde projection radiale 53 de la tige de piston 5, la phase de piquage n'est pas totalement terminée. Ceci est visible sur les figures 6 et 11. A ce moment-là, la surface avant de la seconde projection radiale 53 va faire tourner davantage les organes pivotant 7 en poussant sur leurs secondes projections 72. Ceci va encore davantage tendre ou charger les éléments élastiques 8, qui vont donc opposer une force croissante à la rotation des organes pivotants 7. Ceci génère une force de « freinage » ou « d'amortissement ». Ceci génère un amortissement en fin de piquage, en diminuant la force exercée par la tige de piston 5 sur la seringue S, ce qui améliore grandement le confort de l'utilisateur et évite d'endommager la collerette de la seringue S. Bien entendu, les forces du ressort d'actionnement 6 et des éléments élastiques 8 sont choisies de telle sorte que le piquage n'est qu'amortit sans être stoppé.

Au fur et à mesure que les organes pivotants 7 tournent lors de la phase de piquage, les éléments élastiques 8 se tendent de plus en plus. Simultanément, les axes mobiles 78 des organes pivotants 7 se rapprochent progressivement des axes fixes 79. Le dispositif est ajusté avantageusement pour générer un couple maximal au moment de (ou juste avant) la fin de la phase de piquage. Le point neutre dans lequel les axes mobiles et fixes sont alignés peut donc être atteint au moment de (ou juste avant) la fin de la phase de piquage.

Lorsque les organes pivotants 7 et les éléments élastiques 8 sont dans la position neutre, représentée sur les figures 7 et 12, la tige de piston est toujours sollicitée axialement par le ressort d'actionnement 6. Cette position neutre n'est donc pas stable, et le système bascule automatiquement de l'état de freinage du piquage vers l'état d'amplification du début de l'injection. Eventuellement, la troisième projection 73 peut être disposée par rapport à la seconde projection 72 de telle sorte que les organes pivotants 7 effectuent juste après le passage de la position neutre une légère rotation sous l'effet des éléments élastiques 8 tendus. Ceci peut permettre de générer un son audible lorsque ladite troisième projection 73 va heurter la seconde projection radiale 53 de la tige de piston, pour indiquer à l'utilisateur le début de la phase d'injection.

Lorsque l'aiguille atteint sa position de piquage avec une insertion complète de l'aiguille, la phase d'injection est déclenchée, ce qui est représenté sur les figures 8 et 13. L'extrémité avant 55 de la tige de piston va alors pousser sur le piston sous l'effet de la force exercée par le ressort d'actionnement 6. Pendant toute la phase d'injection, la tige de piston 5 coulisse à l'intérieur de la seringue S en poussant le piston de celle-ci sous l'effet du ressort 6. Le produit est ainsi distribué à travers l'aiguille.

Au début de la phase d'injection, la troisième projection 73 de chaque organe pivotant 7 va donc venir en contact de la surface arrière de la seconde projection radiale 53. Comme en fin de phase de piquage, le couple exercé par le système est maximal juste après la position neutre, et les éléments élastiques 8 tendus sollicitent donc fortement les organes pivotants 7 en rotation. Ceci a pour effet d'amplifier la force du ressort d'actionnement 6 en début de phase d'injection. Cette amplification augmente la force exercée par la tige de piston 5 sur le piston, et permet ainsi de garantir le décollement du piston de sa position de repos, sans avoir à augmenter la force du ressort d'actionnement 6. En effet, la résistance maximale lors de la phase d'injection est créée au moment du décollement du piston. Une fois que l'injection a débutée, les frottements du piston dans la seringue S, la viscosité du produit à injecter et la résistance du passage étroit de l'aiguille sont inférieurs et ne nécessitent donc plus la même force du ressort d'actionnement 6.

Comme visible sur les figures 9 et 14, les organes pivotants 7 se désengrènent de la tige de piston après une course d'injection relativement petite de la tige de piston 5, typiquement de quelques millimètres, par exemple environ 4 mm. A partir de ce désengrènement, le système devient inactif, et l'injection du produit se poursuit normalement. Il est envisageable d'adapter le système de réglage de force pour qu'il puisse amplifier l'effort exercé sur le piston pendant une partie de course d'injection plus grande, par exemple environ 20 mm, voire pendant la totalité de la course d'injection, notamment avec des réservoirs dont la dimension axiale serait réduite.

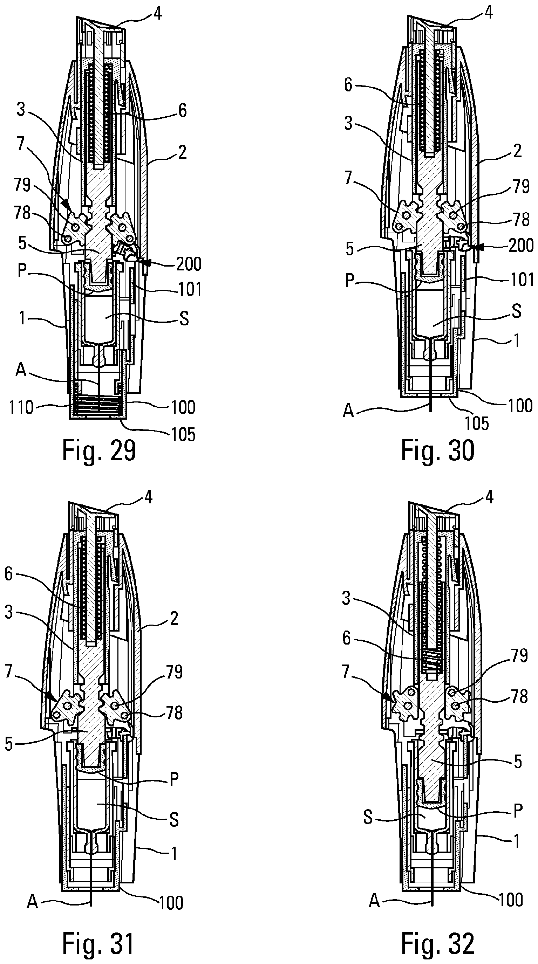

Les figures 29 à 32 illustrent un autre mode de réalisation avantageux de l'autoinjecteur dans lequel il n'y a pas de piquage automatique, ou auto-piquage. Il est à noter que ces figures ne sont que schématiques, et non limitatives d'un tel mode de réalisation. Le ressort d'actionnement 6 ne réalise dans ce mode de réalisation que l'injection en déplaçant la tige de piston 5, et donc le piston P, entre la position de repos et la position d'injection. Ici, le piquage est réalisé manuellement, au moyen du manchon actionneur 100. Dans ce mode de réalisation, la seringue S est donc fixe par rapport au corps de l'autoinjecteur.

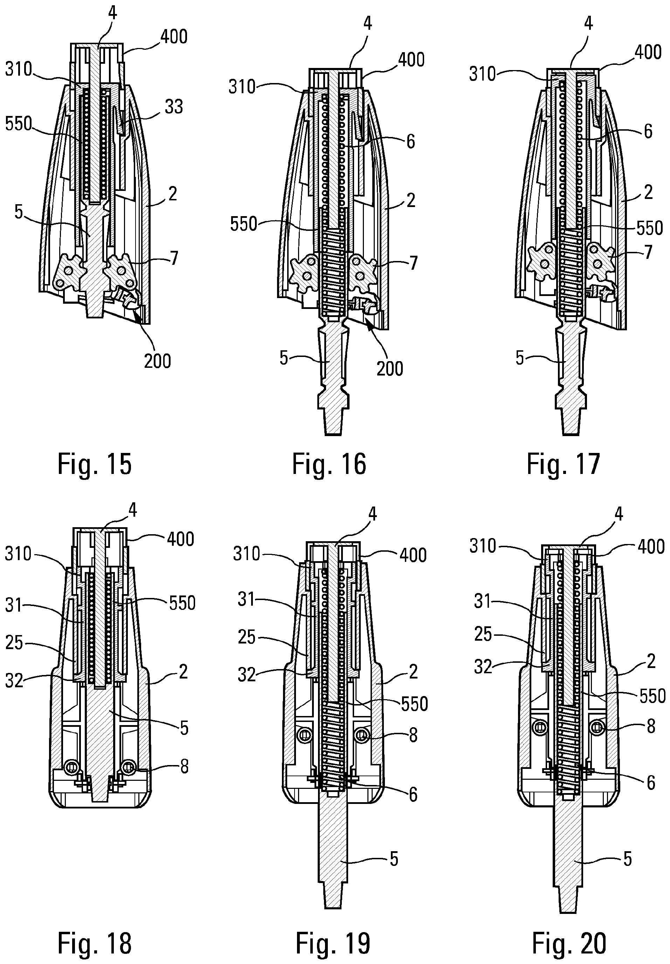

Dans la position de repos illustrée sur la figure 29, le manchon actionneur 100, sollicité par son ressort 110, entoure l'aiguille A de la seringue S. lorsque l'utilisateur veut actionner l'autoinjecteur, il place la surface d'extrémité axiale 105 du manchon actionneur 100 contre le site d'injection, et il appuie sur l'autoinjecteur. Le manchon actionneur 100 va alors coulisser axialement vers l'intérieur du corps inférieur 1, exposant ainsi l'aiguille A de la seringue S qui va alors pénétrer dans le corps de l'utilisateur, comme visible sur la figure 20. L'utilisateur peut alors actionner le bouton axial 4 pour déplacer la tige de piston 5, et le système de réglage de force devient alors actif comme décrit précédemment, en exerçant, via les organes pivotants 7, une force d'amplification F2 sur la tige de piston 5 en début d'injection, comme illustré sur la figure 31. La figure 32 montre les organes pivotants 7 désengrenés de la tige de piston 5, la fin de l'injection se poursuivant alors sans action desdits organes pivotants. En variante, on pourrait envisager de faire agir les organes pivotants pendant toute la phase d'injection.

Dans ce mode de réalisation, le système de réglage de force n'exerce donc que la force d'amplification F2 en début d'injection, et n'intervient pas pendant le piquage.

Selon l'invention, l'autoinjecteur comporte un indicateur visuel et/ou sonore 300 pour indiquer à l'utilisateur la fin de l'injection. Cet indicateur comporte une partie d'indication qui va venir coopérer avec au moins une fenêtre de visualisation en fin d'injection, pour réaliser une indication visuelle. Avantageusement, l'indicateur fournit aussi une indication sonore en plus de l'indication visuelle susmentionnée. L'invention qui sera décrite plus en détails ci-après s'applique autant au mode de réalisation avec auto-piquage décrit en références aux figures 1 à 14 qu'au mode de réalisation sans auto-piquage, décrit en référence aux figures 29 à 32.

Les figures 15 à 22 illustrent un premier mode de réalisation de l'invention, dans lequel l'indicateur est formé par ledit manchon 3.

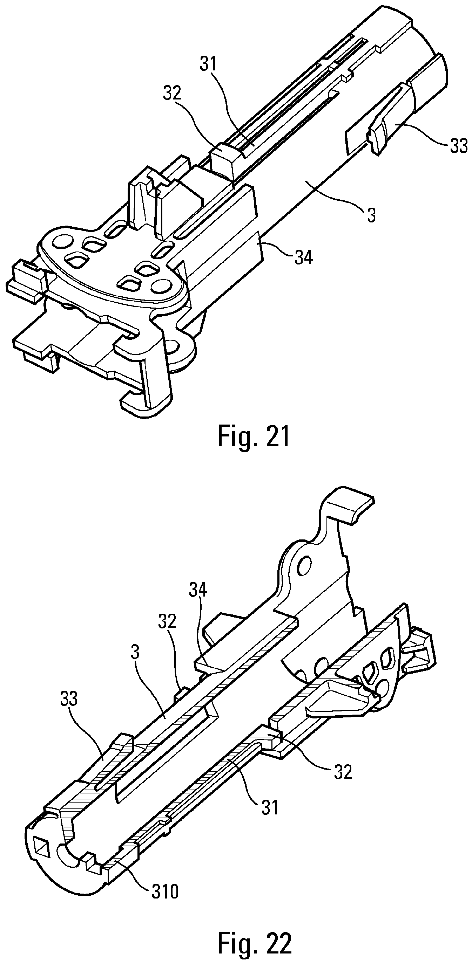

Les figures 21 et 22 montrent en détail la structure dudit manchon 3. Dans ce mode de réalisation, la partie d'indication est formée par une extrémité axiale 310 dudit manchon 3, qui va venir coopérer en fin d'injection avec la ou les fenêtres de visualisation, formée(s) dans le bouton axial 4, comme visible sur les figures 15 à 20. Avantageusement, il y a plusieurs fenêtres de visualisation, par exemple deux, trois ou quatre, réparties autour du bord périphérique axial dudit bouton axial 4. L'extrémité axiale 310 du manchon 3 forme donc une projection axiale, monobloc avec le manchon 3, qui vient se positionner face aux fenêtres de visualisation 400 en fin d'injection.

Le manchon 3 comporte au moins une, de préférence deux, patte(s) axiale(s) 31 radialement déformable(s) et pourvue d'une tête 32. Cette tête 32 coopère avec une partie du corps supérieur 2, en l'occurrence un manchon interne 25 dudit corps supérieur 2. Chaque patte axiale 31 est radialement déformable vers l'intérieur, et est avant et en cours d'actionnement empêchée de se déformer par la tige de piston 5, en particulier un manchon creux 550 de ladite tige de piston 5 qui reçoit le ressort d'actionnement 6. Lors de l'injection, la tige de piston 5 se déplace axialement par rapport audit manchon 3, et en fin d'injection, la tige de piston ne bloque plus lesdites pattes axiales 31. Celles-ci peuvent alors se déformer radialement vers l'intérieur, comme visible sur la figure 20. Ainsi, la tête 32 n'est plus retenue par le manchon interne 25, et le manchon 3 peut alors se déplacer axialement par rapport au corps supérieur 2 sous l'effet du ressort 6, dans la direction opposée à la direction de déplacement de la tige de piston 5 pendant l'actionnement. De ce fait, l'extrémité axiale 310 va venir coopérer avec les fenêtres de visualisation 400 du bouton axial 4.

Une indication sonore est avantageusement fournie pendant ce déplacement axial du manchon 3 par rapport au corps supérieur 2, par un épaulement 34 formé sur le manchon 3, visible sur la figures 21 et 22, et qui va venir heurter une partie du corps supérieur 2, en particulier ledit manchon interne 25, générant ainsi un son audible par l'utilisateur.

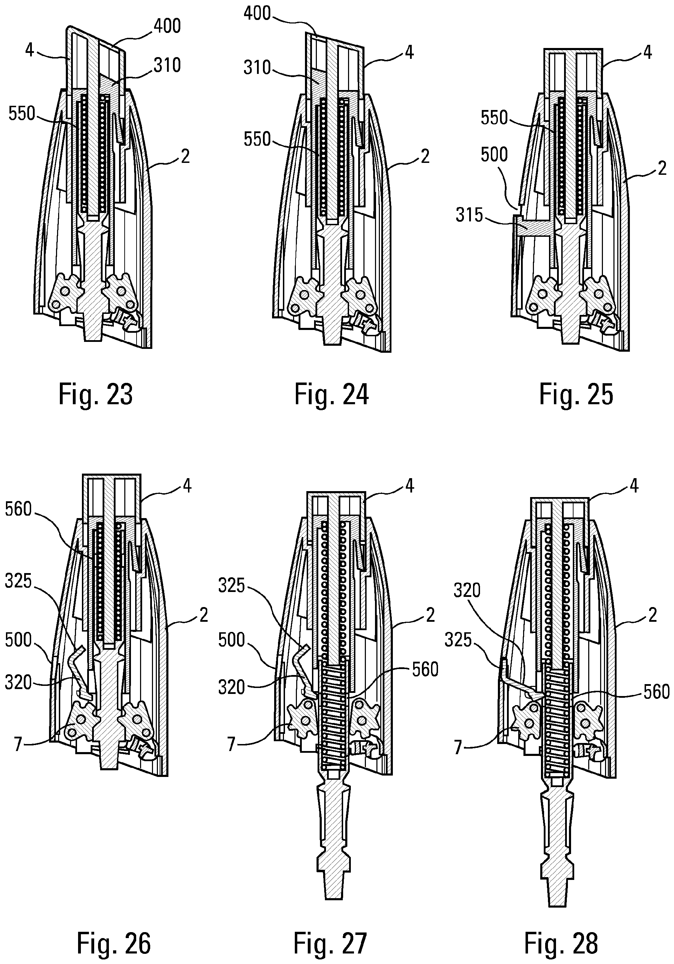

Les figures 23 à 25 illustrent trois variantes de réalisation.

Dans les exemples des figures 23 et 24, la paroi d'extrémité axiale du bouton axial 4 est inclinée, et le bouton axial 4 ne comporte qu'une seule fenêtre de visualisation 400. Dans l'exemple de la figure 23, cette fenêtre de visualisation est disposée dans la partie basse de ladite paroi inclinée (dans la position de la figure 23) alors que dans l'exemple de la figure 24, elle est disposée dans la partie haute. La projection d'extrémité axiale 310 du manchon 3 est adaptée en conséquence, pour avoir une forme appropriée pour coopérer avec ladite fenêtre de visualisation 400.

Dans l'exemple de la figure 25, une fenêtre de visualisation 500 n'est pas formée dans le bouton axial 4 mais dans le corps, en l'occurrence le corps supérieur 2. Le manchon 3 comporte dans ce cas une projection axiale 315 qui s'étend latéralement par rapport audit manchon, et qui va venir coopérer avec ladite fenêtre 500 en fin d'injection.

Les figures 26 à 28 illustrent un autre mode de réalisation, dans lequel l'indicateur n'est pas une partie intégrante du manchon 3 mais est monté pivotant sur celui-ci. La fenêtre de visualisation 500 est formée dans le corps supérieur 2. Un élément d'indication pivotant 320 est ainsi monté pivotant sur ledit manchon 3, à proximité des organes pivotants 7. Une partie d'extrémité 325 dudit élément d'indication pivotant 320 forme alors ladite partie d'indication de l'indicateur. Avantageusement, ledit élément d'indication pivotant 320 est entrainé en rotation par lesdits organes pivotants 7, en fin d'injection. Pour ce faire, la tige de piston 5 et notamment le manchon creux 550 peut comporter une ouverture 560. En cours d'injection, les organes pivotants 7 se désengrènent de la tige de piston 5, mais les ressorts 8 peuvent garder une certaine tension sollicitant lesdits organes pivotant en rotation. Pendant la fin de l'injection, les organes pivotants glissent donc contre la tige de piston 5. Lorsque l'ouverture 560 de la tige de piston 5 arrive au niveau desdits organes pivotants, comme visible sur la figure 27, ceux-ci peuvent faire une petite rotation et par là entrainer l'élément d'indication pivotant 320 en rotation, comme illustré sur la figure 28, pour permettre à la partie d'indication 325 de coopérer avec ladite fenêtre

Bien entendu, d'autres variantes de réalisation sont aussi envisageables.

Typiquement, des forces F1 d'amortissement et/ou F2 d'amplification de l'ordre de 30N sont possibles. Bien entendu, d'autres valeurs d'amortissement et de freinage peuvent être obtenues en choisissant de manière appropriée les éléments élastiques 8 et en dimensionnant de manière appropriée les organes pivotants 7.

La présente invention s'applique à des dispositifs utilisés notamment pour les traitements des maladies auto-immunes, par exemple du type arthrites rhumatoïdes, scléroses multiples, maladie de Crohn, pour les traitements contre le cancer, pour les traitements antiviraux, par exemple du type hépatites, pour les traitements contre le diabète, pour les traitements contre l'anémie ou pour les traitements de crises, par exemple en cas de choc anaphylactique.

Bien que la présente invention ait été décrite en référence à plusieurs modes de réalisation avantageux, il est entendu que diverses modifications sont possibles pour l'homme du métier sans sortir du cadre de la présente invention tel que défini par les revendications annexées.

## Revendications

1. Autoinjecteur comportant un corps (1, 2), un réservoir (S) contenant du produit fluide et comportant un piston et une aiguille, tel qu'une seringue pré-remplie, ledit autoinjecteur comportant une tige de piston (5) adaptée à coopérer avec le piston dudit réservoir (S), ladite tige de piston (5) étant déplaçable entre une position de repos et une position d'injection dans laquelle ladite tige de piston (5) a déplacé le piston du réservoir (S) pour injecter le produit fluide à travers l'aiguille, un ressort d'actionnement (6) étant prévu pour solliciter ladite tige de piston (5) vers sa position d'injection, l'autoinjecteur comprenant un système de réglage de force (7, 8) adapté à exercer au moins une force (F2) sur ladite tige de piston (5), ladite force (F2) s'ajoutant à la force exercée par ledit ressort d'actionnement (6) sur ladite tige de piston (5) en début d'injection pour amplifier la force exercée sur ledit piston par ladite tige de piston (5) en début d'injection, ledit système de réglage de force (7, 8) comprenant deux organes pivotants (7) coopérant avec ladite tige de piston (5), lesdits organes pivotants (7) étant reliés entre eux par deux éléments élastiques (8), ledit corps (1, 2) comportant un manchon (3), lesdits organes pivotants (7) étant monté pivotants sur ledit manchon (3) autour d'axes fixes (79), **caractérisé en ce que** ledit autoinjecteur comprend un indicateur visuel et/ou sonore (300) adapté à indiquer à l'utilisateur la fin de l'injection, ledit indicateur étant formé par et/ou fixé sur ledit manchon (3) et comportant une partie d'indication (310, 315; 325) déplaçable et/ou déformable par rapport audit corps (1, 2) pour coopérer, après la fin de l'injection, avec au moins une fenêtre de visualisation (400; 500) dudit autoinjecteur, lesdits éléments élastiques (8) étant fixés auxdits organes pivotants (7) au niveau d'axes mobiles parallèles (78), tels que des tiges ayant deux bords latéraux, formés sur lesdits organes pivotants (7), dans lequel, lorsque la tige de piston (5) se déplace vers sa position d'injection, lesdits axes mobiles (78) sont disposés en arrière desdits axes fixes (79) dans le sens de déplacement de ladite tige de piston (5), lesdits éléments élastiques chargés (8), en début de déplacement de la tige de piston (5) vers sa position d'injection, faisant tourner lesdits organes pivotants (7) de telle manière à détendre lesdits éléments élastiques (8), créant ainsi une force d'amplification (F2) en début d'injection.

2. Autoinjecteur selon la revendication 1, dans lequel chaque organe pivotant (7) comprend plusieurs projections (71, 72, 73) adaptées à coopérer avec plusieurs projections radiales (52, 53) de la tige de piston (5).

3. Autoinjecteur selon l'une quelconque des revendications précédentes, dans lequel lors du piquage, la tige de piston (5) coopère avec le piston du réservoir (S) pour déplacer ledit réservoir (S) par rapport au corps (1, 2).

4. Autoinjecteur selon l'une quelconque des revendications précédentes, dans lequel, avant injection, ladite tige de piston (5) est d'abord déplacée par ledit ressort d'actionnement (6) entre ladite position de repos et une position de piquage, dans laquelle ladite tige de piston (5) a déplacé ledit réservoir (S) par rapport audit corps (1, 2) pour réaliser le piquage.

5. Autoinjecteur selon la revendication 4, dans lequel lors du piquage, la tige de piston (5) coopère avec le piston du réservoir (S) pour déplacer ledit réservoir (S) par rapport au corps (1, 2).

6. Autoinjecteur selon la revendication 4 ou 5, dans lequel, lorsque la tige de piston (5) se déplace de sa position de repos vers sa position de piquage, lesdits axes mobiles (78) sont disposés en avant desdits axes fixes (79) dans le sens de déplacement de ladite tige de piston (5), ladite tige de piston (5), en fin de déplacement vers sa position de piquage, faisant tourner lesdits organes pivotants (7) de telle manière à charger lesdits éléments élastiques (8), créant ainsi une force de freinage (F1) en fin de piquage, ladite force (F1) étant opposée à la force exercée par ledit ressort d'actionnement (6) sur ladite tige de piston (5) en fin de piquage pour diminuer la force exercée sur ledit réservoir (S) par ladite tige de piston (5) en fin de piquage.

7. Autoinjecteur selon l'une quelconque des revendications précédentes, dans lequel ledit autoinjecteur est actionné par un bouton axial (4).

8. Autoinjecteur selon la revendication 7, dans lequel ladite au moins une fenêtre de visualisation (400) est formée dans ledit bouton axial (4).

9. Autoinjecteur selon la revendication 8, dans lequel ledit bouton axial (4) comporte une seule fenêtre de visualisation (400) disposée dans une paroi d'extrémité axiale inclinée dudit bouton axial (4).

10. Autoinjecteur selon la revendication 8, dans lequel ledit bouton axial (4) comporte une pluralité de fenêtres de visualisation (400), réparties autour du bord d'extrémité axial extérieur dudit bouton axial (4).

11. Autoinjecteur selon l'une quelconque des revendications 1 à 7, dans lequel ladite au moins une fenêtre de visualisation (500) est formée dans ledit corps (1, 2).

12. Autoinjecteur selon l'une quelconque des revendications précédentes, dans lequel ledit indicateur est formé par ledit manchon (3) comportant une projection axiale monobloc (310, 315) formant ladite partie d'indication de l'indicateur.

13. Autoinjecteur selon l'une quelconque des revendications 1 à 11, dans lequel ledit indicateur est formé par un élément d'indication pivotant (320) monté pivotant sur ledit manchon (3), une partie d'extrémité (325) dudit élément d'indication pivotant (320) formant ladite partie d'indication de l'indicateur.

14. Autoinjecteur selon la revendication 13, dans lequel ledit élément d'indication pivotant (320) est entrainé en rotation par lesdits organes pivotants (7), en fin d'injection.

15. Autoinjecteur selon l'une quelconque des revendications précédentes, dans lequel ledit manchon (3) comporte au moins une patte axiale (31) radialement déformable et coopérant avec ledit corps (1, 2), la déformation de ladite patte axiale (31) étant bloquée par ladite tige de piston (5) avant et pendant le déplacement de ladite tige de piston (5), bloquant ainsi tout déplacement axial dudit manchon (3) par rapport audit corps (1, 2), ladite tige de piston (5) libérant ledit blocage en fin d'injection, de sorte que ladite patte axiale (31) se déforme radialement vers l'intérieur, permettant le déplacement axial dudit manchon (3) par rapport audit corps (1, 2) pour réaliser une indication visuelle et/ou sonore.

## Patentansprüche

1. Autoinjektor umfassend einen Körper (1, 2), einen Vorratsbehälter (S), der ein Flüssigprodukt enthält, und einen Kolben und eine Nadel, wie beispielsweise eine Fertigspritze, umfasst, wobei der Autoinjektor eine Kolbenstange (5) umfasst, die derart ausgelegt ist, dass sie mit dem Kolben des Vorratsbehälters (S) zusammenwirkt, wobei die Kolbenstange (5) zwischen einer Ruhestellung und einer Injektionsstellung bewegbar ist, in welcher die Kolbenstange (5) den Kolben des Vorratsbehälters (S) derart verschoben hat, dass das Flüssigprodukt durch die Nadel hindurch injiziert wird, wobei eine Antriebsfeder (6) zur Vorspannung der Kolbenstange (5) in Richtung ihrer Injektionsstellung vorgesehen ist, wobei der Autoinjektor ein Krafteinstellsystem (7, 8) umfasst, das derart ausgelegt ist, dass es mindestens eine Kraft (F2) auf die Kolbenstange (5) ausübt, wobei die Kraft (F2) zusätzlich zu der zu Beginn des Injektionsvorgangs von der Antriebsfeder (6) auf die Kolbenstange (5) ausgeübten Kraft wirkt und dadurch die zu Beginn des Injektionsvorgangs von der Kolbenstange (5) auf den Kolben ausgeübte Kraft verstärkt, wobei das Krafteinstellsystem (7, 8) zwei schwenkbare Elemente (7) umfasst, die mit der Kolbenstange (5) zusammenwirken, wobei die schwenkbaren Elemente (7) über zwei elastische Elemente (8) miteinander verbunden sind, wobei der Körper (1, 2) eine Hülse (3) umfasst, wobei die schwenkbaren Elemente (7) um feste Achsen (79) schwenkbar an der Hülse (3) angebracht sind, **dadurch gekennzeichnet, dass** der Autoinjektor eine visuelle und/oder akustische Anzeige (300) umfasst, die derart ausgelegt ist, dass sie dem Benutzer das Ende des Injektionsvorgangs anzeigt, wobei die Anzeige durch die Hülse (3) gebildet und/oder an dieser angebracht ist und einen Anzeigeabschnitt (310, 315; 325) umfasst, welcher in Bezug auf den Körper (1, 2) verschiebbar und/oder verformbar ist, so dass er nach dem Ende des Injektionsvorgangs mit mindestens einem Sichtfenster (400; 500) des Autoinjektors zusammenwirkt, wobei die elastischen Elemente (8) an den schwenkbaren Elementen (7) an parallelen beweglichen Achsen (78), zum Beispiel Stiften mit zwei Seitenkanten, befestigt sind, die an den schwenkbaren Elementen (7) ausgebildet sind, wobei bei Verschiebung der Kolbenstange (5) in Richtung ihrer Injektionsstellung die beweglichen Achsen (78) in Verschieberichtung der Kolbenstange (5) gesehen hinter den festen Achsen (79) angeordnet sind, wobei die belasteten elastischen Elemente (8) zu Beginn der Verschiebung der Kolbenstange (5) in Richtung ihrer Injektionsstellung die schwenkbaren Elemente (7) derart drehen, dass die elastischen Elemente (8) entspannt werden, wodurch zu Beginn des Injektionsvorgangs eine Verstärkungskraft (F2) erzeugt wird.

2. Autoinjektor nach Anspruch 1, bei dem jedes schwenkbare Element (7) eine Vielzahl von Vorsprüngen (71, 72, 73) aufweist, die derart ausgelegt sind, dass sie mit einer Vielzahl von radialen Vorsprüngen (52, 53) der Kolbenstange (5) zusammenwirken.

3. Autoinjektor nach einem der voranstehenden Ansprüche, bei dem die Kolbenstange (5) beim Einstechvorgang mit dem Kolben des Vorratsbehälters (S) zusammenwirkt und dadurch den Vorratsbehälter (S) in Bezug auf den Körper (1, 2) verschiebt.

4. Autoinjektor nach einem der voranstehenden Ansprüche, bei dem vor der Injektion die Kolbenstange (5) zunächst von der Antriebsfeder (6) zwischen der Ruhestellung und einer Einstechstellung verschoben wird, in welche die Kolbenstange (5) den Vorratsbehälter (S) in Bezug auf den Körper (1, 2) verschoben hat, um den Einstechvorgang durchzuführen.

5. Autoinjektor nach Anspruch 4, bei dem beim Einstechvorgang die Kolbenstange (5) mit dem Kolben des Vorratsbehälters (S) zusammenwirkt, um den Vorratsbehälter (S) in Bezug auf den Körper (1, 2) zu verschieben.

6. Autoinjektor nach Anspruch 4 oder 5, bei dem, wenn sich die Kolbenstange (5) aus ihrer Ruhestellung in ihre Einstechstellung verschiebt, die beweglichen Achsen (78) in Verschieberichtung der Kolbenstange (5) gesehen vor den festen Achsen (79) angeordnet sind, wobei die Kolbenstange (5) am Ende der Verschiebung in Richtung ihrer Einstechstellung die schwenkbaren Elemente (7) derart dreht, dass die elastischen Elemente (8) belastet werden, wodurch eine Bremskraft (F1) am Ende des Einstechvorgangs erzeugt wird, wobei diese Kraft (F1) der Kraft entgegengesetzt ist, die von der Antriebsfeder (6) am Ende des Einstechvorgangs auf die Kolbenstange (5) ausgeübt wird, um die von der Kolbenstange (5) am Ende des Einstechvorgangs auf den Vorratsbehälter (S) ausgeübte Kraft zu verringern.

7. Autoinjektor nach einem der voranstehenden Ansprüche, bei dem die Betätigung des Autoinjektors über einen axialen Knopf (4) erfolgt.

8. Autoinjektor nach Anspruch 7, bei dem das mindestens eine Sichtfenster (400) in dem axialen Knopf (4) ausgebildet ist.

9. Autoinjektor nach Anspruch 8, bei dem der axiale Knopf (4) ein einziges Sichtfenster (400) aufweist, das in einer schrägen axialen Stirnwand des axialen Knopfes (4) angeordnet ist.

10. Autoinjektor nach Anspruch 8, bei dem der axiale Knopf (4) eine Vielzahl von Sichtfenstern (400) aufweist, die an der äußeren axialen Randkante des axialen Knopfes (4) herum verteilt angeordnet sind.

11. Autoinjektor nach einem der Ansprüche 1 bis 7, bei dem das mindestens eine Sichtfenster (500) in dem Körper (1, 2) ausgebildet ist.

12. Autoinjektor nach einem der voranstehenden Ansprüche, bei dem der Indikator durch die Hülse (3) gebildet wird, die einen einstückigen axialen Vorsprung (310, 315) umfasst, welcher den Anzeigeabschnitt der Anzeige bildet.

13. Autoinjektor nach einem der Ansprüche 1 bis 11, bei dem die Anzeige durch ein schwenkbares Anzeigeelement (320) gebildet wird, das schwenkbar an der Hülse (3) angebracht ist, wobei ein Endabschnitt (325) des schwenkbaren Anzeigeelements (320) den Anzeigeabschnitt der Anzeige bildet.

14. Autoinjektor nach Anspruch 13, bei dem das schwenkbare Anzeigeelement (320) am Ende des Injektionsvorgangs von den schwenkbaren Elementen (7) gedreht wird.

15. Autoinjektor nach einem der voranstehenden Ansprüche, bei dem die Hülse (3) mindestens einen radial verformbaren axialen Ansatz (31) aufweist, der mit dem Körper (1, 2) zusammenwirkt, wobei die Verformung dieses axialen Ansatzes (31) vor und bei der Verschiebung der Kolbenstange (5) von der Kolbenstange (5) blockiert wird, wodurch folglich auch jedwede axiale Verschiebung der Hülse (3) in Bezug auf den Körper (1, 2) blockiert wird, wobei am Ende des Injektionsvorgangs die Kolbenstange (5) die Blockierung löst, wodurch sich der axiale Ansatz (31) radial nach innen verformt, was die axiale Verschiebung der Hülse (3) in Bezug auf den Körper (1, 2) ermöglicht, um eine optische und/oder akustische Anzeige zu erzeugen.

## Claims

1. An autoinjector comprising a body (1, 2), and a reservoir (S) containing fluid and including a piston and a needle, such as a pre-filled syringe, said autoinjector further comprising a piston rod (5) that is adapted to co-operate with the piston of said reservoir (S), said piston rod (5) being movable between a rest position and an injection position in which said piston rod (5) has moved the piston of the reservoir (S) so as to inject the fluid through the needle, an actuator spring (6) being provided so as to urge said piston rod (5) towards its injection position, the autoinjector further comprising a force-adjustment system (7, 8) that is adapted to exert at least one force (F2) on said piston rod (5), said force (F2) adding to the force exerted by said actuator spring (6) on said piston rod (5) at the beginning of injection, so as to amplify the force exerted on said piston by said piston rod (5) at the beginning of injection, said force-adjustment system (7, 8) comprising two pivot members (7) co-operating with said piston rod (5), said pivot members (7) being connected together by two resilient elements (8), said body (1, 2) including a sleeve (3), said pivot members (7) being pivotally mounted on said sleeve (3) to pivot about stationary pins (79), **characterized in that** said autoinjector includes a visual and/or audible indicator (300) adapted to inform the user that injection has ended, said indicator being formed by and/or fastened to said sleeve (3) and including an indication portion (310, 315; 325) that is movable and/or deformable relative to said body (1, 2) so as to co-operate, after the end of injection, with at least one viewing window (400; 500) of said autoinjector, said resilient elements (8) being fastened to said pivot members (7) via parallel movable pins (78), such as rods having two side edges, formed on said pivot members (7), wherein, when the piston rod (5) moves towards its injection position, said movable pins (78) are arranged behind said stationary pins (79) in the travel direction of said piston rod (5), said loaded resilient elements (8), at the beginning of travel of the piston rod (5) towards its injection position, causing said pivot members (7) to pivot in such a manner as to relax said resilient elements (8), thereby creating an amplification force (F2) at the beginning of injection.

2. Autoinjector according to claim 1, wherein each pivot member (7) includes a plurality of projections (71, 72, 73) adapted to co-operate with a plurality of radial projections (52, 53) of the piston rod (5).

3. Autoinjector according to any preceding claim, wherein during pricking, the piston rod (5) co-operates with the piston of the reservoir (S) so as to move said reservoir (S) relative to the body (1, 2).

4. Autoinjector according to any preceding claim, wherein, prior to injection, said piston rod (5) is initially moved by said actuator spring (6) between said rest position and a pricking position, in which said piston rod (5) has moved said reservoir (S) relative to said body (1, 2) so as to perform pricking.

5. Autoinjector according to claim 4, wherein during pricking, the piston rod (5) co-operates with the piston of the reservoir (S) so as to move said reservoir (S) relative to the body (1, 2).

6. Autoinjector according to claim 4 or 5, wherein, when the piston rod (5) moves from its rest position towards its pricking position, said movable pins (78) are arranged in front of said stationary pins (79) in the travel direction of said piston rod (5), said piston rod (5), at the end of travel towards its pricking position, causing said pivot members (7) to pivot so as to load said resilient elements (8), thereby creating a braking force (F1) at the end of pricking, said force (F1) being opposed to the force exerted by said actuator spring (6) on said piston rod (5) at the end of pricking so as to decrease the force exerted on said reservoir (S) by said piston rod (5) at the end of pricking.

7. Autoinjector according to any preceding claim, wherein said autoinjector is actuated by an axial button (4).

8. Autoinjector according to claim 7, wherein said at least one viewing window (400) is formed in said axial button (4).

9. Autoinjector according to claim 8, wherein said axial button (4) includes a single viewing window (400) arranged in a sloping axial end wall of said axial button (4).

10. Autoinjector according to claim 8, wherein said axial button (4) includes a plurality of viewing windows (400), distributed around the outer axial end edge of said axial button (4).

11. Autoinjector according to any one of claims 1 to 7, wherein said at least one viewing window (500) is formed in said body (1, 2).

12. Autoinjector according to any preceding claim, wherein said indicator is formed by said sleeve (3) including a single-piece axial projection (310, 315) forming said indication portion of the indicator.

13. Autoinjector according to any one of claims 1 to 11, wherein said indicator is formed by a pivotable indicator element (320) that is pivotally mounted on said sleeve (3), an end portion (325) of said pivotable indicator element (320) forming said indication portion of the indicator.

14. Autoinjector according to claim 13, wherein said pivotable indicator element (320) is pivoted by said pivot members (7), at the end of injection.

15. Autoinjector according to any preceding claim, wherein said sleeve (3) includes at least one axial tab (31) radially deformable and that co-operates with said body (1, 2), the deforming of said axial tab (31) being prevented by said piston rod (5) prior to and during movement of said piston rod (5), thus blocking any axial movement of said sleeve (3) relative to said body (1, 2), said piston rod (5) releasing said blocking at the end of injection, such that said axial tab (31) deforms radially inwards, enabling said sleeve (3) to move axially relative to said body (1, 2) so as to provide a visual and/or audible indication.
